(19) Europäisches Patentamt
European Patent Office
Office européen des brevets

(11) EP 4 480 506 A1

(12) EUROPEAN PATENT APPLICATION
published in accordance with Art. 153(4) EPC

(43) Date of publication:
**25.12.2024 Bulletin 2024/52**

(21) Application number: **22930650.1**

(22) Date of filing: **12.12.2022**

(51) International Patent Classification (IPC):
***A61L 24/00*** *(2006.01)* ***A61L 24/06*** *(2006.01)*
***A61L 24/08*** *(2006.01)* ***C08F 251/00*** *(2006.01)*
***C08F 222/20*** *(2006.01)* ***C08F 220/58*** *(2006.01)*
***C08F 2/48*** *(2006.01)* ***C08F 299/00*** *(2006.01)*
***C08G 81/00*** *(2006.01)*

(86) International application number:
**PCT/CN2022/138355**

(87) International publication number:
**WO 2023/169028 (14.09.2023 Gazette 2023/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.03.2022 CN 202210221842**

(71) Applicant: **Genera Translation Biomedicine (Shenzhen) Co., Ltd**
**Shenzhen, Guangdong 518000 (CN)**

(72) Inventors:
- **WU, Decheng**
**Shenzhen, Guangdong 518055 (CN)**
- **PAN, Zheng**
**Shenzhen, Guangdong 518055 (CN)**
- **ZHANG, Chong**
**Shenzhen, Guangdong 518055 (CN)**

(74) Representative: **Manitz Finsterwald Patent- und Rechtsanwaltspartnerschaft mbB**
**Martin-Greif-Strasse 1**
**80336 München (DE)**

# (54) HEMOSTATIC SPONGE AND PREPARATION METHOD THEREFOR

(57) The present invention relates to the field of medicine. Specifically provided are a hemostatic sponge and a preparation method therefor. Components for preparing the hemostatic sponge comprise, by mass, 3-30 parts of a double bond-containing compound, 1-10 parts of chitosan, and 0.1-15 parts of a photoinitiator. Compared with existing commercial products, the hemostatic sponge of the present invention has a higher water absorption percentage and water absorption rate, and can quickly swell after absorbing water. When applied to a wound for hemostasis, the hemostatic sponge can quickly block the wound and achieve a hemostatic effect.

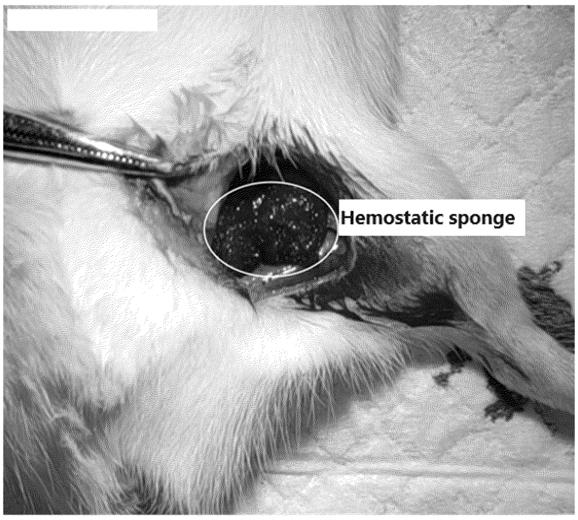


Fig. 3

EP 4 480 506 A1

## Description

### Technical Field

The present invention relates to the field of medicine, and specifically relates to a hemostatic sponge and a preparation method therefor.

### Background Art

Damage to and massive bleeding from human tissue may occur on battlefields and in accidents, and timely on-site emergency treatment can greatly increase the survival rate of patients. At present, common hemostatic materials include hemostatic bandages, hemostatic powders, hemostatic gels, expandable hemostatic sponges, etc. Hemostatic bandages cannot fit well on irregularly shaped wounds and cannot block deep wounds for hemostasis. Hemostatic powders and hemostatic gels, when used on wounds with massive bleeding, can hardly adhere to the wound surface since they are washed by the bleeding blood, which limits their applications. Expandable hemostatic sponges, thanks to its ability to rapidly expand and block wounds, have become an excellent option for dealing with bleeding from deep wounds in emergency situations. On the other hand, infection may seriously affect the healing of wounds. Therefore, the development of an expandable hemostatic sponge that can quickly stop bleeding and also has an antimicrobial effect would be of great significance in on-site emergency treatment. However, existing hemostatic sponges have a poor expandability and a low compression strength, and the preparation method therefor are complex. Further, subject to pore-forming mechanisms, materials that can be selected for the existing hemostatic sponges are limited.

### Summary of the Invention

According to a first aspect, in an embodiment, there is provided a hemostatic sponge, the components for preparing the hemostatic sponge comprising, by mass, 3 to 30 parts of a double bond-containing compound, 1 to 10 parts of chitosan, and 0.1 to 15 parts of a photoinitiator.

According to a second aspect, in an embodiment, there is provided a method for preparing the hemostatic sponge according to the first aspect, comprising:

a precursor solution preparation step, comprising mixing the components according to the formula amounts, and dissolving same in a solvent to prepare a precursor solution;
a depressurizing treatment step, comprising performing depressurizing treatment on the precursor solution to obtain an expanded precursor solution;
an illumination step, comprising illuminating the expanded precursor solution to obtain a formed sponge;
a soaking step, comprising soaking the formed sponge in a salt solution to obtain a soaked sponge; and
a freeze-drying step, comprising freeze-drying the soaked sponge to obtain the hemostatic sponge.

In accordance with the hemostatic sponge and the method for preparing same according to the above embodiments, the raw materials for the hemostatic sponge of the present invention are simple, and all of the raw materials are highly biocompatible.

In an embodiment, the hemostatic sponge of the present invention has higher water absorptivity and water absorption rate compared with existing products, and can quickly expand after absorbing water. When applied to a wound for hemostasis, the hemostatic sponge can quickly block the wound to stop bleeding.

In an embodiment, the chitosan in the hemostatic sponge of the present invention can adsorb red blood cells to induce intrinsic blood coagulation, thus reducing the hemostasis time for the wound.

In an embodiment, the hemostatic sponge of the present invention has such a high strength that satisfies the requirement for stopping bleeding from the wound by means of pressing.

### Brief Description of the Drawings

Fig. 1 shows pictures showing expansion, due to water absorption, of the sponge prepared in Example 1, which has been compressed;
Fig. 2 shows a stress-strain diagram for the sponge prepared in Example 1 which was subjected to 10 cycles of compression to 80% strain;
Fig. 3 shows a picture showing the effect of the sponge prepared in Example 1 in stopping bleeding from femoral

arteries of a rat;
Fig. 4 shows a picture showing the external shape of the sponge prepared in Example 2; and
Fig. 5 shows an SEM picture showing adsorption of red blood cells by the sponge prepared in Example 2.

## Detailed Description of the Embodiments

The present application will be further illustrated below by detailed description of the embodiments with reference to the accompanying drawings, in which like elements in different embodiments are indicated with like reference numerals. In the following embodiments, many details are described so that the present application will be better understood. However, those skilled in the art can readily recognize that some of the features may be omitted, or replaced by other elements, materials, or methods, depending on different situations. In some cases, some operations related to the present application are not shown or described in this specification, so as to avoid overwhelming the core part of the present application with excessive description. Detailed description of these relevant operations is not necessary for those skilled in the art, who can have a complete knowledge of the relevant operations in light of the disclosure in the description and the general technical knowledge in the art.

Additionally, the characteristics, operations or features described in the specification can be combined in any suitable manner to form various embodiments. Moreover, the steps or actions in the description of the method may also be switched or adjusted in sequence in a manner that is obvious to those skilled in the art. Therefore, the various sequences in the description and the drawings are merely for the purpose of clearly describing a particular embodiment and are not intended to be required, unless it is otherwise specified that a specific sequence must be followed.

The serial numbers per se, such as "first", "second", etc., designated herein for components, are only used for distinguishing the described objects and do not represent any sequence or have any technical meaning. As used herein, "connected" or "coupled" includes both direct and indirect connection (coupling), unless otherwise specified.

Herein, "PEG" can be used interchangeable with "polyethylene glycol" .

According to a first aspect, in an embodiment, there is provided a hemostatic sponge, the components for preparing the hemostatic sponge comprising, by mass, 3 to 30 parts of a double bond-containing compound, 1 to 10 parts of chitosan, and 0.1 to 15 parts of a photoinitiator.

The double bond-containing compound crosslinks upon illumination to form a chemical network that maintains the shape of the sponge, such that the sponge can still retain an intact porous structure after being restored from a vacuum degree to the atmospheric pressure. The chitosan soaked in the salt solution forms a physical network. The photoinitiator is used to initiate the crosslinking of the double bond-containing compound to form the chemical network and maintain the shape of the sponge.

In an embodiment, the part by mass of the double bond-containing compound includes, but is not limited to, 3 parts, 4 parts, 5 parts, 6 parts, 7 parts, 8 parts, 9 parts, 10 parts, 11 parts, 12 parts, 13 parts, 14 parts, 15 parts, 16 parts, 17 parts, 18 parts, 19 parts, 20 parts, 21 parts, 22 parts, 23 parts, 24 parts, 25 parts, 26 parts, 27 parts, 28 parts, 29 parts, 30 parts, etc.

In an embodiment, the part by mass of the chitosan includes, but is not limited to, 1 part, 2 parts, 3 parts, 4 parts, 5 parts, 6 parts, 7 parts, 8 parts, 9 parts, 10 parts, etc.

In an embodiment, the part by mass of the photoinitiator includes, but is not limited to, 0.1 parts, 0.2 parts, 0.3 parts, 0.4 parts, 0.5 parts, 0.6 parts, 0.7 parts, 0.8 parts, 0.9 parts, 1 part, 2 parts, 3 parts, 4 parts, 5 parts, 6 parts, 7 parts, 8 parts, 9 parts, 10 parts, 11 parts, 12 parts, 13 parts, 14 parts, 15 parts, etc.

In an embodiment, the components for preparing the hemostatic sponge comprise, by mass, 3 to 20 parts of the double bond-containing compound, 1 to 10 parts of the chitosan, and 0.7 to 15 parts of the photoinitiator.

In an embodiment, the double bond-containing compound includes compounds represented by the following general formula I:

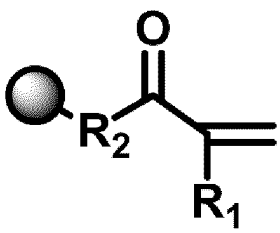

I ;

where $R_1$ is hydrogen (-H) or methyl ($-CH_3$) ;
$R_2$ is oxygen (-O-) or imino (-NH-) ;

“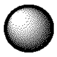”

to which $R_2$ is connected represents a main chain. The main chain is that chain in a branch chain (side chain) structure-containing polymer chain which has the greatest number of chain segments.

In an embodiment, the double bond-containing compound includes at least one of the compounds represented by the following general formula II to general formula IX:

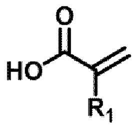

II

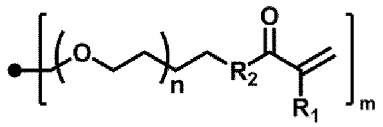

III

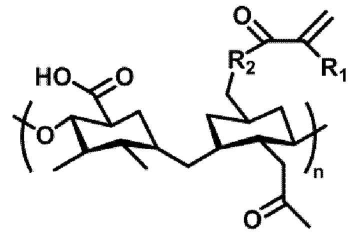

IV

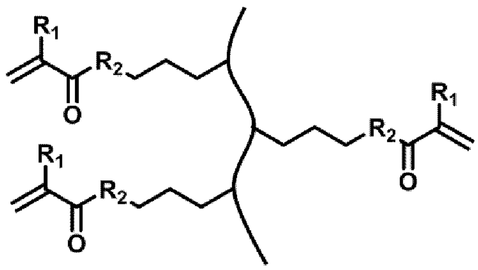

V

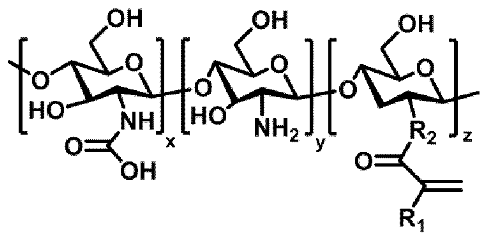

VI

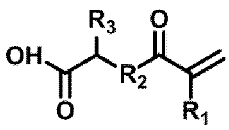

VII

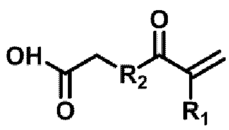

VIII

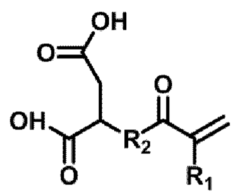

IX

;

In general formula II to general formula IX, $R_1$ is hydrogen (-H) or methyl ($-CH_3$), $R_2$ is oxygen (-O-) or imino (-NH-) ;

formula II is double bond-containing acrylic acid or methacrylic acid;
formula III is double bond-containing polyethylene glycol (PEG-AA), where m represents the number of polyethylene glycol arms, which may specifically be an integer of 2 to 8; "•" represents a carbon atom located in the middle of the multi-arm polyethylene glycol acrylate or multi-arm polyethylene glycol methacrylate and used to connect multiple identical polyethylene glycol acrylate or polyethylene glycol methacrylate chain segments; and n represents the number of repeating units of polyethylene glycol, which may specifically be an integer of 28 to 112, and correspond to formula III having a molecular weight of 2 to 40 kDa;
formula IV is double bond-containing hyaluronic acid, where n represents the number of repeating units of hyaluronic acid, which may specifically be an integer of 25 to 2500, and correspond to formula IV having a molecular weight of 10

to 1000 kDa;
formula V is double bond-containing gelatin (Gel-MA);
formula VI is double bond-containing chitosan, where x, y, and z represent the number of respective repeating units of chitosan, which may specifically be an integer of 62 to 6200, and correspond to formula VI having a molecular weight of 10 to 1000 kDa;
formula VII is a double bond compound whose terminal group contains a carboxyl group, $R_3$ represents a side chain group, which may be any group, such as hydrogen (-H) as shown in formula VIII and $-CH_2-COOH$ as shown in formula IX, wherein if $R_2$ is imino (-NH-), then formula VII may be a double bond-functionalized amino acid;
if $R_2$ in formula VIII is imino, then formula VIII is N-(meth)acryloyl glycine; if $R_2$ in formula IX is imino, then formula IX is (meth)acryloyl glutamic acid.

In an embodiment, the double bond-containing polyethylene glycol (PEG) represented by formula III includes, but is not limited to, at least one of polyethylene glycol diacrylate (formula III, m=2), eight-arm polyethylene glycol acrylate (formula III, m=8), and four-arm polyethylene glycol acrylate (formula III, m=4).
In an embodiment, in formula VII, $R_3$ includes, but is not limited to, hydrogen, $-CH_3$, -OH, $-CH_2-COOH$, $-CH_2CH_3$, $-CH_2-CH(CH_3)_2$, $-CH_2-C_6H_5$, $-CH_2-C_6H_4-OH$, and $-CH_2-SH$.
In an embodiment, the chitosan is water-soluble chitosan. Any chitosan which is soluble in water is suitable for used in the present invention.
In an embodiment, the chitosan has a molecular weight of about 5 to 20 kDa (kilo Daltons), including but not limited to, 5 kDa, 6 kDa, 7 kDa, 8 kDa, 9 kDa, 10 kDa, 11 kDa, 12 kDa, 13 kDa, 14 kDa, 15 kDa, 16 kDa, 17 kDa, 18 kDa, 19 kDa, and 20 kDa.
In an embodiment, the photoinitiator includes, but is not limited to at least one of photoinitiator 651 (also known as benzil dimethyl ether, a, a-dimethoxy-a-phenylacetophenone, abbreviated as DMPA, CAS number: 24650-42-8), photoinitiator 1173 (2-hydroxy-2-methyl-1-phenyl-1-propanone, abbreviated as HMPP, CAS number: 7473-98-5), photoinitiator 2959 (2-hydroxy-2-methyl-1- [4-(2-hydroxyethoxy)phenyl]-1-propanone, CAS number: 106797-53-9), TPO (2,4,6-trimethylbenzoyl-diphenylphosphine oxide, CAS number: 75980-60-8), $\alpha$-ketoglutaric acid (2-oxoglutaric acid, CAS number: 328-50-7), LAP (lithium phenyl-2,4,6-trimethylbenzoyl phosphite, CAS number: 85073-19-4), etc.
In an embodiment, the components for preparing the hemostatic sponge further include a salt.
In an embodiment, the components for preparing the hemostatic sponge further include an aqueous solution comprising the salt.
In an embodiment, the salt includes, but is not limited to, at least one of NaCl, $CaCl_2$, KCl, $NaH_2PO_4$, $KH_2PO_4$, $Na_2CO_3$, $Na_2SO_4$, $Na_2HPO_4$, $K_2HPO_4$, $KH_2PO_4$, $Na_3Cit$, and $Na_3PO_4$.
In an embodiment, the salt solution may be a saturated aqueous solution.
In an embodiment, the concentration of the salt in the salt solution may be 15wt% to 40wt%.
In an embodiment, the salt solution may be a PBS buffer solution.
According to a second aspect, in an embodiment, there is provided a method for preparing the hemostatic sponge according to the first aspect, comprising:

a precursor solution preparation step, comprising mixing the components according to the formula amounts, and dissolving same in a solvent to prepare a precursor solution;
a depressurizing treatment step, comprising performing depressurizing treatment on the precursor solution to obtain an expanded precursor solution;
an illumination step, comprising illuminating the expanded precursor solution to obtain a formed sponge;
a soaking step, comprising soaking the formed sponge in a salt solution to obtain a soaked sponge; and
a freeze-drying step, comprising freeze-drying the soaked sponge to obtain the hemostatic sponge.

In an embodiment, in the precursor solution preparation step, the solvent is water.
In an embodiment, in the precursor solution preparation step, the mass of the solvent is 1 to 100 times, preferably 10 to 100 times the mass of the chitosan.
In an embodiment, the mass of the solvent may be 5 times or more the mass of the double bond-containing compound monomer, such that after illumination, the material can set, and after being restored from a vacuum degree to the atmospheric pressure, can maintain the shape.
In an embodiment, in the precursor solution preparation step, the mass of the solvent is 10 to 100 times the mass of the chitosan. If the usage amount of the solvent is too high, then it is impossible to form good bubbles; if the usage amount of the solvent is too low, then the chitosan cannot be dissolved. There is no specific requirement for the usage amount of the photoinitiator.
In an embodiment, in the precursor solution preparation step, the precursor solution is stirred until uniform bubbles form, prior to performing the depressurizing treatment step.

In an embodiment, in the depressurizing treatment step, the vacuum degree in a container containing the precursor solution is 20 to 100 mbar. The vacuum degree includes, but is not limited to, 20 mbar, 30 mbar, 40 mbar, 50 mbar, 60 mbar, 70 mbar, 80 mbar, 90 mbar, 100 mbar, etc.

In an embodiment, in the illumination step, the wavelength of the light used in illumination is 200 to 450 nm. The wavelength includes, but is not limited to, 200 nm, 210 nm, 220 nm, 230 nm, 240 nm, 250 nm, 260 nm, 270 nm, 280 nm, 290 nm, 300 nm, 310 nm, 320 nm, 330 nm, 340 nm, 350 nm, 360 nm, 370 nm, 380 nm, 390 nm, 400 nm, 410 nm, 420 nm, 430 nm, 440 nm, 450 nm, etc.

In an embodiment, in the illumination step, the duration of illumination is 1 to 100 min. The duration of illumination includes, but is not limited to, 1 min, 2 min, 3 min, 4 min, 5 min, 6 min, 7 min, 8 min, 9 min, 10 min, 20 min, 30 min, 40 min, 50 min, 60 min, 70 min, 80 min, 90 min, 100 min, etc.

In an embodiment, in the soaking step, the salt solution includes, but is not limited to, at least one of NaCl aqueous solution, $CaCl_2$ aqueous solution, KCl aqueous solution, $NaH_2PO_4$ aqueous solution, $KH_2PO_4$ aqueous solution, $Na_2CO_3$ aqueous solution, $Na_2SO_4$ aqueous solution, $Na_2HPO_4$ aqueous solution, $K_2HPO_4$ aqueous solution, $Na_3Cit$ (sodium citrate) aqueous solution, $Na_3PO_4$ aqueous solution, and PBS buffer solution. Soaking in the salt solution allows the chitosan to form a physical network, which increases the strength and shape restoration performance of the sponge. Without the soaking, the sponge is prone to be brittle and absorbs water slowly.

In an embodiment, in the soaking step, the duration of soaking in the salt solution is 1 to 200 min. The soaking duration includes, but is not limited to, 1 min, 2 min, 3 min, 4 min, 5 min, 6 min, 7 min, 8 min, 9 min, 10 min, 20 min, 30 min, 40 min, 50 min, 60 min, 70 min, 80 min, 90 min, 100 min, 110 min, 120 min, 130 min, 140 min, 150 min, 160 min, 170 min, 180 min, 190 min, 200 min, etc.

In an embodiment, in the soaking step, after soaking in the salt solution is completed, soaking is performed using water prior to performing the freeze-drying step.

In an embodiment, in the soaking step, after soaking in the salt solution is completed, the sponge is taken out of the salt solution, allowed to stand for 1 to 100 h, and is then soaked using water. The standing duration includes, but is not limited to, 1 h, 2 h, 3 h, 4 h, 5 h, 6 h, 7 h, 8 h, 9 h, 10 h, 20 h, 30 h, 40 h, 50 h, 60 h, 70 h, 80 h, 90 h, 100 h, etc. During standing, the salt solution on/in the sponge solution drains off.

In an embodiment, in the soaking step, the duration of soaking in water is 1 to 100 h. The soaking duration includes, but is not limited to, 1 h, 2 h, 3 h, 4 h, 5 h, 6 h, 7 h, 8 h, 9 h, 10 h, 20 h, 30 h, 40 h, 50 h, 60 h, 70 h, 80 h, 90 h, 100 h, etc.

In an embodiment, the present invention provides a preparation method for, and a use of, a highly expandable hemostatic sponge system which absorbs blood fast. The hemostatic sponge is prepared using a double bond-containing compound and chitosan. Specific synthesis steps involve producing a porous structure by a simple method of controlling vacuum degree, forming a chemical network by means of light induced crosslinking of the double bond-containing compound so as to maintain the shape of the sponge, and inducing the chitosan to form a physical crosslinking network by soaking in a salt solution so as to impart the sponge with an excellent shape restoration ability. In exerting a hemostasis effect, the sponge which has been compressed can rapidly absorb a large amount of blood and undergo volume expansion. After restoring to the original shape, the sponge can block the bleeding wound and prevent itself from being washed away by the outflowing blood, thereby achieving effective blocking of the bleeding wound. The hemostatic sponge has a good biocompatibility, and can be made in various shapes and sizes according to needs so as to be suitable for use with different wounds.

In an embodiment, the present invention provides a porous structure-containing and highly swellable hemostatic sponge system which is prepared by means of vacuuming, and a use thereof.

In an embodiment, an aim of the present invention is to provide a highly swellable hemostatic sponge system which is prepared by a simple method, and a use thereof. The hemostatic sponge has the characteristics of high swelling rate, fast blood absorption, high strength, shape memory, adjustable performance, etc.

The present invention provides a highly swellable hemostatic sponge system, which is prepared mainly from a double bond-containing compound and chitosan. The double bond-containing compound and chitosan are dissolved in water and stirred to form bubbles. The precursor solution containing uniform bubbles is poured into a container, and the container is vacuumed. After the vacuum degree stabilizes, the precursor solution is illuminated to induce polymerization of the double bond-containing compound so as to form a first layer of chemical network. Afterwards, the system is soaked in a salt solution such that the chitosan undergoes physical crosslinking so as to form a second layer of network. The first layer of chemical network can maintain the shape of the sponge and increase the strength of the sponge, and can absorb the water in the blood. The chitosan physical crosslinking network can increase the shape restoration ability of the sponge, such that a wound can be blocked by means of expansion of the sponge itself. Moreover, the chitosan can agglutinate red blood cells in the blood, enhance the adhesion, activation, and aggregation of blood platelets, and promote hemostasis. In addition, the positively charged chitosan can inhibit microbes and prevent wound infection.

In an embodiment, the raw materials for preparing the hemostatic sponge comprise, by mass, 3 to 20 parts of the double bond-containing compound, 1 to 10 parts of the chitosan, 0.1 to 15 parts of the photoinitiator, and 100 parts of water.

In an embodiment, the structural general formula of the double bond-containing compound used in the present

invention is I, where $R_1$ is hydrogen (-H) or methyl ($-CH_3$), $R_2$ is oxygen (-O-) or imino (-NH-). The double bond-containing compound may specifically be (meth)acrylic acid, (meth)acrylamide, double bond-functionalized polyethylene glycol (PEG-AA), a double bond-modified amino acid, double bond-functionalized gelatin (Gel-MA), double bond-modified chitosan, double bond-modified sodium alginate, and double bond-modified hyaluronic acid, etc.

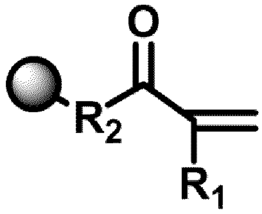

I

In some embodiments, the structure of the double bond-containing compound may be any one of formula II to formula IX, where $R_1$ is hydrogen (-H) or methyl ($-CH_3$), $R_2$ is oxygen (-O-) or imino (-NH-). If formula II is (meth)acrylic acid, formula III is double bond-containing PEG (PEG-AA), where m represents the number of PEG arms, which may specifically be 2 to 8; n represents the number of repeating units of PEG, which may specifically be 28-112, and correspond to a molecular weight of 2 to 40 kDa; formula IV is double bond-containing hyaluronic acid, where n represents the number of repeating units of hyaluronic acid, which may specifically be 25 to 2500, and correspond to a molecular weight of 10 to 1000 kDa; formula V is double bond-containing gelatin (Gel-MA); formula VI is double bond-containing chitosan, where x, y, and z represent the number of respective repeating units of chitosan, which may specifically be 62 to 6200, and correspond to a molecular weight of 10 to 1000 kDa; formula VII is a double bond compound whose terminal group contains a carboxyl group, $R_3$ represents a side chain group, which may be any group, such as hydrogen (-H) as shown in formula VIII and $-CH_2-COOH$ as shown in formula IX, wherein if $R_2$ is imino (-NH-), then formula VII may be a double bond-functionalized amino acid; wherein if $R_2$ in formula VIII is imino, then formula VIII is N-(meth)acryloyl glycine; if $R_2$ in formula IX is imino, then formula IX is (meth)acryloyl glutamic acid.

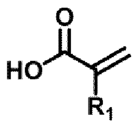

II

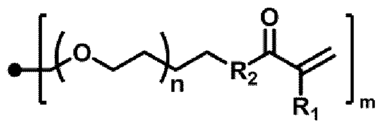

III

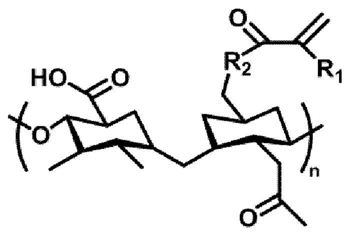

IV

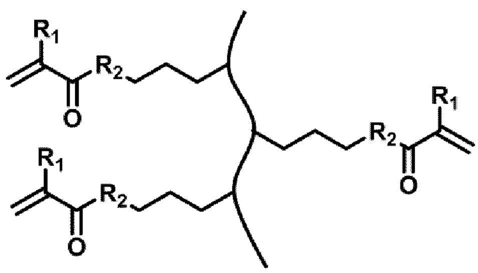

V

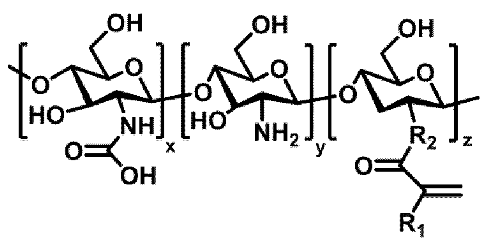

VI

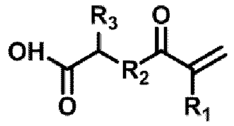

VII

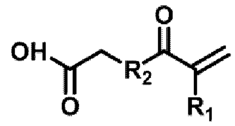

VIII

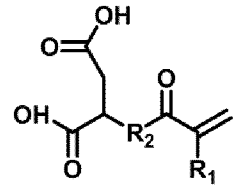

IX

In some embodiments, the chitosan used in the present invention is water-soluble chitosan.

In some embodiments, the photoinitiator used in the present invention may be photoinitiator 651, photoinitiator 1173, photoinitiator 2959, TPO, α-ketoglutaric acid, LAP, etc.

In some embodiments, the preparation method for the hemostatic sponge provided in the present invention comprises: dissolving 3 to 30 parts of the double bond-containing compound, 1 to 10 parts of chitosan, and 0.1 to 15 parts of the photoinitiator in 100 parts of water to prepare a precursor solution; stirring the precursor solution using a stirrer to form uniform and fine bubbles; taking a certain amount of the stirred precursor solution and placing same in a container, and subjecting the container to depressurizing treatment at a vacuum degree of 20 to 100 mbar such that the precursor solution expands; after the pressure stabilizes and the volume of the precursor solution no longer changes, illuminating the container with light having a wavelength of 200 to 450 nm for a duration of 1 to 100 min such that the precursor solution gels; taking a sample, and placing same in at least one solution selected from NaCl aqueous solution, $CaCl_2$ aqueous solution, KCl aqueous solution, $NaH_2PO_4$ aqueous solution, $KH_2PO_4$ aqueous solution, $Na_2CO_3$ aqueous solution, $Na_2SO_4$ aqueous solution, $Na_2HPO_4$ aqueous solution, $K_2HPO_4$ aqueous solution, $Na_3Cit$ aqueous solution, $Na_3PO_4$ aqueous solution, PBS buffer solution, etc. to soak for 1 to 200 min; removing the sample, allowing water to drain off, and allowing the sample the stand for 1 to 100 h; and after the standing, placing the sample in pure water, soaking same for 1 to 100 h, and freeze-drying same to obtain the sponge.

In an embodiment, the hemostatic sponge prepared in the present invention has a porosity of 85% to 99%, a density of 0.01 to 0.1g/$cm^3$, a saturated water absorptivity of 2000% to 50000%, a time period required for saturated water absorption of 1 to 300 s , and a compressive strength of 30 to 200 kPa at 80% strain.

In an embodiment, the hemostatic sponge prepared in the present invention has shape restoration performance. Its shape after compression in a dry state can be maintained, and after absorbing water, it can rapidly restore to the shape before compression.

In an embodiment, the raw materials for the hemostatic sponge of the present invention are simple, and all of the raw materials are highly biocompatible.

In an embodiment, the preparation method for the hemostatic sponge of the present invention is simple, has a short preparation period, and can achieve performance adjustment.

In an embodiment, the hemostatic sponge of the present invention has higher water absorptivity and water absorption rate compared with existing products, and can quickly expand after absorbing water. When applied to a wound for hemostasis, the hemostatic sponge can quickly block the wound to stop bleeding.

In an embodiment, the chitosan in the hemostatic sponge of the present invention can adsorb red blood cells to induce intrinsic blood coagulation, thus reducing the hemostasis time for the wound.

In an embodiment, the hemostatic sponge of the present invention has such a high strength that satisfies the requirement for stopping bleeding from the wound by means of pressing.

In the following examples and comparative examples, the non-double bond-containing chitosan used has a molecular weight of about 10 kDa. For example, in Example 1, the chitosan having a mass of 0.05 g is non-double bond-containing chitosan. The same applies in other examples and comparative examples.

Example 1

0.15 g of polyethylene glycol diacrylate (formula III, m=2) having a molecular weight of 10 kDa, 0.01 g photoinitiator 2959, and 0.05 g of chitosan were weighed. All of the materials were dissolved in 1 g of ultrapure water, and were stirred using a stirrer to form uniform bubbles. All of the materials were filled into a vacuum container, which was vacuumed to 50 mbar. After the pressure stabilized, the materials were illuminated with light of 350 nm for 30 min to allow forming. The formed mass was removed, soaked in a saturated $Na_2SO_4$ aqueous solution for 20 min, and was then removed and allowed to stand for 12 h. After the standing, the resulting sample was placed in ultrapure water for 12 h. The sample was removed from the ultrapure water, placed in a freeze-drying machine, and freeze-dried in an environment of -50 to -45°C and 0.05 to 0.15 mbar, thereby obtaining a sponge. In subsequent examples and comparative examples, if there was the freeze-drying step, the freeze-drying conditions were the same as those in this example.

Test of water absorption performance: A piece of the dried sponge having a height of about 10 mm, a diameter of

about 13 cm, and a mass of about 0.04 g was taken, and was weighed to obtain initial mass $W_0$. The sponge was immersed in ultrapure water. Every one hour, the sponge was taken out, and was weighed after residual water on its surface was wiped off. For each weighing, the sponge was weighed five times, and an average value was obtained and recorded as the weighed mass for that weighing. The weighed sponge was immersed in pure water to allow swelling again. If in three consecutive weighings, the mass no longer changed, then the mass was recorded as final mass $W_t$. The water absorption performance of the sponge was calculated according to the following formula:

$$SR = \frac{W_t - W_0}{W_0} \times 100\%$$

SR: water absorptivity of the sponge;
W : final mass of the sponge after absorbing water and becoming expanded; and
$W_0$: initial mass of the sponge before absorbing water.

For each group, five samples were taken to conduct the test, and an average value was obtained and recorded as the final water absorptivity. The water absorptivity of the sponge measured according to the above method was 5800%.

Test of water absorption time: A piece of the dried sponge having a height of about 10 mm, a diameter of about 13 cm, and a mass of about 0.04 g was taken, and was compressed to obtain compressed sponge having a height of 2 to 3 mm. The sponge was placed in water, and timekeeping was started; after the sponge became completely expanded, the timekeeping was stopped. The time taken was recorded as the water absorption time of the sponge, as shown in Fig. 1. For each group, five samples were taken to conduct the test, and an average value was obtained and recorded as the final water absorption time. The water absorption time of the sponge measured by the above method was 7.2 s.

Test of water retention performance: A dried piece of sponge having a height of about 10 mm was taken, and was weighed to obtain the initial weight $W_0$. The sponge was immersed in ultrapure water to allow it to sufficiently swell until saturation. The sponge was taken out and centrifuged at 500 r/min for 3 min, and was then weighed to obtain the mass $W_1$. The water retention rate of the sponge was calculated according to the following formula:

$$WRV = \frac{W_1 - W_0}{W_0} \times 100\%$$

WRV: water retention rate of the sponge;
$W_1$: the mass of the sponge after absorbing water and being centrifuged; and
$W_0$: the initial mass of the sponge before absorbing water.

For each group, five samples were taken to conduct the test, and an average value was obtained and recorded as the final water retention rate. The water retention rate of the sponge measured by the above method was 2200%.

Test of mechanical performance: The sponge after absorbing water and being centrifuged was cut to obtain a sample having a diameter to height ratio of 1:(0.33 to 0.67). A universal testing machine was used to cyclically compress the sample to 80% strain at a speed of 5 mm/min. The highest compression strength δobtained was recorded.

Samples for compression were obtained by cutting the sponge. For each group, five samples were made, and an average value was obtained. The values obtained for the samples for compression which were within the diameter to height ratio range of 1:(0.33 to 0.67) were valid.

For each group, five samples were taken to conduct the test, and an average value was obtained and recorded as the final strength. The strength of the sponge measured by the above method was 67 kPa. Fig. 2 shows a stress-strain curve for 10 cycles. It can be seen that the loss was marginal, indicating that the sponge had a good strength and a good shape restoration performance.

Measurement of porosity and density:

A piece of dried sponge was taken, and was weighed to obtain the initial mass $W_s$. The sponge was placed in a container, and a certain amount of ethanol was added such that the ethanol immersed the sponge (such that the ethanol could still immerge the sponge after the sponge had sufficiently absorbed ethanol). The total mass $W_a$ of the container, the ethanol, and the sponge was weighed. After ultrasonication for 2 min, the sponge was taken out, and the total mass $W_b$ of the container and the remaining ethanol was weighed. This sponge (that is, the foregoing dried sponge, the initial mass being $W_s$) was contained using a measuring cylinder, and the total mass was weighed to be Wi; the sponge was put into the

measuring cylinder, such that ethanol reached the original graduation, and the total weight $W_2$ was weighed. The porosity of the sponge was calculated according to the following formula:

$$P = \frac{w_a - w_b - w_s}{(w_a - w_b) - (w_2 - w_1)} \times 100\%.$$

The density was calculated according to the following formula:

$$\rho = \frac{\rho_E \times w_s}{(w_a - w_b) - (w_2 - w_1)} \times 100\%;$$

$\rho_E$: density of ethanol. In each measurement, the density of all the ethanol in that measurement was obtained by dividing the mass of the ethanol by the volume of the ethanol, and the value was close to 0.79 g/cm$^3$.

For each group, five samples were taken to conduct the test, and average values were obtained and recorded as the final porosity and density. Measured by the above method, the porosity of the sponge was 89%, and the density of the sponge was 0.031g/cm$^3$.

Measurement of hemostasis time and blood loss amount.

Test of truncation and hemostasis of femoral arteries of a rat: Left and right femoral arteries of the rat were exposed. A 6 mm tissue sampler or puncher was used to make a 5 mm-deep cavity at the femoral arteries and surrounding tissue. Then, a compressed sponge was inserted in the cavity. Blood that flowed out was collected using absorbent cotton, and bleeding was observed. The time required until no blood outflow was observed was hemostasis time, and the mass of the blood collected was blood loss amount. The hemostasis effect is as shown in Fig. 3.

For each group, five samples were taken to conduct the test, and average values were obtained and recorded as the final hemostasis time and blood loss amount. Measured by the above method, the hemostasis time was 101 s, and the blood loss amount was 2.31 g.

The tests of performance indexes in subsequent examples and comparative examples were performed in accordance with this example.

Example 2

0.1 g of double bond-containing chitosan having a molecular weight of 10 kDa, 0.008 g of photoinitiator LAP (chemical name: lithium phenyl-2,4,6-trimethylbenzoyl phosphite, CAS number: 85073-19-4), and 0.08 g of chitosan were weighed. All of the materials were dissolved in 1 g of ultrapure water, and were stirred using a stirrer to form uniform bubbles. All of the materials were filled into a vacuum container, which was vacuumed to 40 mbar. After the pressure stabilized, the materials were illuminated with light of 270 nm for 5 min to allow forming. The formed mass was removed, soaked in a 20wt% $Na_3Cit$ aqueous solution (namely, sodium citrate aqueous solution) for 10 min, and was then removed and allowed to stand for 5 h. After the standing, the resulting sample was placed in ultrapure water for 24 h. The sample was removed from the ultrapure water, and was then freeze-dried in a freeze-drying machine, thereby obtaining a sponge (Fig. 4). Measurements of the sponge were: a water absorptivity of 10000%, a water absorption time of 5.8 s, a water retention rate of 3300%, a strength of 52 kPa, a porosity of 87%, a density of 0.055 g/cm$^3$, a hemostasis time of 84 s, and a blood loss amount of 1.92 g. Fig. 5 shows an SEM picture showing adsorption of red blood cells by the sponge. It can be seen from the figure that the sponge prepared could adsorb a large number of red blood cells, and thus could effectively promote the hemostasis process.

Example 3

0.2 g of N-acryloyl glycine, 0.02 g of photoinitiator 651, and 0.02 g of chitosan were weighed. All of the materials were dissolved in 1 g of ultrapure water, and were stirred using a stirrer to form uniform bubbles. All of the materials were filled into a vacuum container, which was vacuumed to 80 mbar. After the pressure stabilized, the materials were illuminated with light of 290 nm for 60 min to allow forming. The formed mass was removed, soaked in a 40wt% NaCl aqueous solution for 50 min, and was then removed and allowed to stand for 3 h. After the standing, the resulting sample was placed in ultrapure water for 30 h. The sample was removed from the ultrapure water, and was then freeze-dried in a freeze-drying machine, thereby obtaining a sponge. Measurements of the sponge were: a water absorptivity of 50000%, a water absorption time of 9.1 s, a water retention rate of 10000%, a strength of 36 kPa, a porosity of 92%, a density of 0.039 g/cm$^3$, a hemostasis time of 79 s, and a blood loss amount of 1.73 g.

Example 4

0.15 g of double bond-containing gelatin having a molecular weight of 40 kDa, 0.15 g of photoinitiator α-ketoglutaric acid, and 0.05 g of chitosan were weighed. All of the materials were dissolved in 1 g of ultrapure water, and were stirred using a stirrer to form uniform bubbles. All of the materials were filled into a vacuum container, which was vacuumed to 30 mbar. After the pressure stabilized, the materials were illuminated with light of 450 nm for 6 min to allow forming. The formed mass was removed, soaked in a 1×PBS solution (namely, a PBS buffer solution) for 70 min, and was then removed and allowed to stand for 10 h. After the standing, the resulting sample was placed in ultrapure water for 20 h. The sample was removed from the ultrapure water, and was then freeze-dried in a freeze-drying machine, thereby obtaining a sponge. Measurements of the sponge were: a water absorptivity of 25000%, a water absorption time of 8.9 s, a water retention rate of 5100%, a strength of 59 kPa, a porosity of 93%, a density of 0.041 g/$cm^3$, a hemostasis time of 186 s, and a blood loss amount of 3.21 g.

Example 5

0.18 g of eight-arm polyethylene glycol acrylate (formula III, m=8) having a molecular mass of 5 kDa, 0.007 g of photoinitiator 1173, and 0.01 g of chitosan were weighed. All of the materials were dissolved in 1 g of ultrapure water, and were stirred using a stirrer to form uniform bubbles. All of the materials were filled into a vacuum container, which was vacuumed to 60 mbar. After the pressure stabilized, the materials were illuminated with light of 200 nm for 70 min to allow forming. The formed mass was removed, soaked in a 20wt% $Na_2CO_3$ aqueous solution for 2 min, and was then removed and allowed to stand for 3 h. After the standing, the resulting sample was placed in ultrapure water for 15 h. The sample was removed from the ultrapure water, and was then freeze-dried in a freeze-drying machine, thereby obtaining a sponge. Measurements of the sponge were: a water absorptivity of 31000%, a water absorption time of 10.2 s, a water retention rate of 7100%, a strength of 67 kPa, a porosity of 92%, a density of 0.029 g/$cm^3$, a hemostasis time of 134 s, and a blood loss amount of 2.97 g.

Example 6

0.18 g of hyaluronic acid having a molecular weight of 200 kDa, 0.02 g of photoinitiator TPO, and 0.075 g of chitosan were weighed. All of the materials were dissolved in 1 g of ultrapure water, and were stirred using a stirrer to form uniform bubbles. All of the materials were filled into a vacuum container, which was vacuumed to 30 mbar. After the pressure stabilized, the materials were illuminated with light of 320 nm for 10 min to allow forming. The formed mass was removed, soaked in a 15wt% $CaCl_2$ aqueous solution for 6 min, and was then removed and allowed to stand for 5 h. After the standing, the resulting sample was placed in ultrapure water for 40 h. The sample was removed from the ultrapure water, and was then freeze-dried in a freeze-drying machine, thereby obtaining a sponge. Measurements of the sponge were: a water absorptivity of 11000%, a water absorption time of 9.9 s, a water retention rate of 2300%, a strength of 78 kPa, a porosity of 89%, a density of 0.041 g/$cm^3$, a hemostasis time of 156 s, and a blood loss amount of 2.44 g.

Example 7

0.03 g of methacryloyl glutamic acid, 0.02 g of photoinitiator 1173, and 0.1 g of chitosan were weighed. All of the materials were dissolved in 1 g of ultrapure water, and were stirred using a stirrer to form uniform bubbles. All of the materials were filled into a vacuum container, which was vacuumed to 30 mbar. After the pressure stabilized, the materials were illuminated with light of 450 nm for 100 min to allow forming. The formed mass was removed, soaked in a saturated $K_2HPO_4$ aqueous solution for 200 min, and was then removed and allowed to stand for 20 h. After the standing, the resulting sample was placed in ultrapure water for 100 h. The sample was removed from the ultrapure water, and was then freeze-dried in a freeze-drying machine, thereby obtaining a sponge. Measurements of the sponge were: a water absorptivity of 1800%, a water absorption time of 5.1 s, a water retention rate of 700%, a strength of 88 kPa, a porosity of 87%, a density of 0.055 g/$cm^3$, a hemostasis time of 199 s, and a blood loss amount of 2.77 g.

Example 8

0.2 g of four-arm polyethylene glycol acrylate (formula III, m=4) having a molecular mass of 20 kDa, 0.05 g of photoinitiator 1173, and 0.08 g of chitosan were weighed. All of the materials were dissolved in 1 g of ultrapure water, and were stirred using a stirrer to form uniform bubbles. All of the materials were filled into a vacuum container, which was vacuumed to 45 mbar. After the pressure stabilized, the materials were illuminated with light of 340 nm for 12 min to allow forming. The formed mass was removed, soaked in a saturated $NaH_2PO_4$ aqueous solution for 5 min, and was then removed and allowed to stand for 12 h. After the standing, the resulting sample was placed in ultrapure water for 40 h. The

sample was removed from the ultrapure water, and was then freeze-dried in a freeze-drying machine, thereby obtaining a sponge. Measurements of the sponge were: a water absorptivity of 6000%, a water absorption time of 11.7 s, a water retention rate of 1500%, a strength of 59 kPa, a porosity of 95%, a density of 0.027 g/cm$^3$, a hemostasis time of 87 s, and a blood loss amount of 1.92 g.

Comparative Example 1

0.15 g of polyethylene glycol diacrylate (formula III, m=2) having a molecular weight of 10 kDa, 0.02 g of photoinitiator 2959, and 0.05 g of chitosan were weighed. All of the materials were dissolved in 1 g of ultrapure water, and were stirred using a stirrer to form uniform bubbles. Vacuuming was not performed (the difference compared with Example 1 lied in that the materials were not vacuumed in a container to expand the sponge bubbles), and directly illumination with light of 350 nm was performed. The formed mass was removed, soaked in a saturated $Na_2SO_4$ aqueous solution for 20 min, and was then removed and allowed to stand for 12 h. After the standing, the resulting sample was placed in ultrapure water for 12 h. The sample was removed from the ultrapure water, and was then freeze-dried in a freeze-drying machine, thereby obtaining a sample without elasticity and incapable of being compressed, which was in essence a freeze-dried hydrogel. Measurements of the sample were: a water absorptivity of 880%, a water absorption time of 3 h, a water retention rate of 720%, and a strength of 840 kPa. Since the sample was a freeze-dried rigid hydrogel, its porosity and density could not be measured, and it could not be used for hemostasis.

Comparative Example 2

0.2 g of N-acryloyl glycine, 0.02 g of photoinitiator 651, and 0.02 g of chitosan were weighed. All of the materials were dissolved in 1 g of ultrapure water, and were not stirred (the difference compared with Example 3 lied in that the materials were not stirred to form uniform bubbles). All of the materials were filled into a vacuum container, which was vacuumed to 80 mbar. After the pressure stabilized, the materials were illuminated with light of 290 nm for 60 min to allow forming. The formed mass was removed, soaked in a 40wt% NaCl aqueous solution for 50 min, and was then removed and allowed to stand for 3 h. After the standing, the resulting sample was placed in ultrapure water for 30 h. The sample was removed from the ultrapure water, and was then freeze-dried in a freeze-drying machine, thereby obtaining a sponge. Measurements of the sponge were: a water absorptivity of 1100%, a water absorption time of 3 h, a water retention rate of 970%, and a strength of 790 kPa. Since the sponge was in essence a freeze-dried rigid hydrogel, its porosity and density could not be measured, and it could not be used for hemostasis.

Comparative Example 3

0.03 g of methacryloyl glutamic acid, 0.02 g of photoinitiator 1173, and 0.1 g of chitosan were weighed. All of the materials were dissolved in 1 g of ultrapure water, and were stirred using a stirrer to form uniform bubbles. All of the materials were filled into a vacuum container, which was vacuumed to 30 mbar. After the pressure stabilized, the materials were illuminated with light of 450 nm for 100 min to allow forming. The formed mass was removed, soaked in a saturated $K_2HPO_4$ aqueous solution for 200 min, and was then removed and allowed to stand for 20 h. The soaked mass was directly freeze-dried in a freeze-drying machine, thereby obtaining a sponge (the difference compared with Example 7 lied in that the formed mass having been soaked in the salt solution was not allowed to stand in water, but was freeze-dried directly). Measurements of the sponge were: a water absorptivity of 900%, a water absorption time of 67 s, a water retention rate of 600%, a strength of 112 kPa, a porosity of 81%, and a density of 0.075 g/cm$^3$. Since the sponge was rigid and swelled slowly, it could not be used for hemostasis.

Comparative Example 4

0.2 g of four-arm polyethylene glycol acrylate (formula III, m=4) having a molecular mass of 20 kDa, 0.05 g of photoinitiator 1173, and 0.08 g of chitosan were weighed. All of the materials were dissolved in 1 g of ultrapure water, and were stirred using a stirrer to form uniform bubbles. All of the materials were filled into a vacuum container, which was vacuumed to 45 mbar. After the pressure stabilized, the materials were illuminated with light of 340 nm for 12 min to allow forming. The formed mass was removed, and was directly freeze-dried in a freeze-drying machine to obtain a sponge (the difference compared with Example 8 lied in that the formed mass was not soaked in a salt solution and water, but was directly freeze-dried). Measurements of the sponge were: a water absorptivity of 7000%, a water absorption time of 8.2 s, a water retention rate of 700%, a strength of 6 kPa, a porosity of 98%, and a density of 0.021 g/cm$^3$. Due to insufficient strength, the sponge could not be used for hemostasis.

Comparative Example 5

0.2 g of four-arm polyethylene glycol acrylate (formula III, m=4) having a molecular mass of 20 kDa, 0.05 g of photoinitiator 1173, and 0.005 g of chitosan were weighed (the difference compared with Example 8 lied in that the amount of chitosan was low). All of the materials were dissolved in 1 g of ultrapure water. Stirring using a stirrer could not form uniform and stable bubbles, and subsequent operations could not be performed.

Comparative Example 6

0.01 g of hyaluronic acid having a molecular mass of 200 kDa (the difference compared with Example 6 lied in that the amount of the double bond-containing compound was low), 0.02 g of photoinitiator TPO, and 0.075 g of chitosan were weighed. All of the materials were dissolved in 1 g of ultrapure water, and were stirred using a stirrer to form uniform bubbles. All of the materials were filled into a vacuum container, which was vacuumed to 30 mbar. After the pressure stabilized, the materials were illuminated with light of 320 nm for 10 min to allow forming. The formed mass was restored to atmospheric pressure. The resulting sponge was broken and could not maintain the original shape, and subsequent operations could not be performed.

The present invention has been described above with reference to specific examples, which are merely intended to aid in the understanding of the present invention and are not intended to limit the present invention thereto. Several simple derivations, variations or substitutions can be made by a person skilled in the art to which the present invention pertains in light of the concept of the present invention.

## Claims

1. A hemostatic sponge, **characterized in that** components for preparing the hemostatic sponge comprise, by mass, 3 to 30 parts of a double bond-containing compound, 1 to 10 parts of chitosan, and 0.1 to 15 parts of a photoinitiator;

   the double bond-containing compound includes at least one of compounds represented by following general formula II to general formula IX:

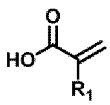

II

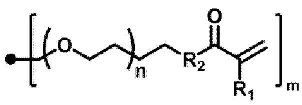

III

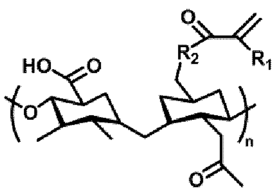

IV

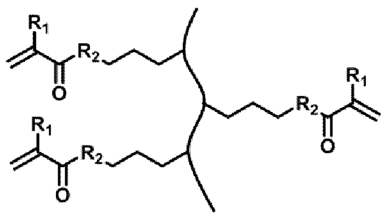

V

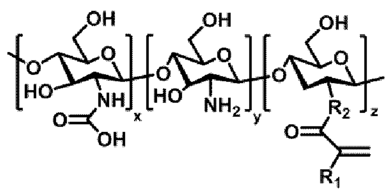

VI

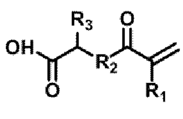

VII

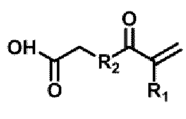

VIII

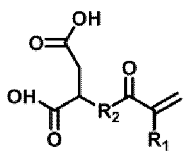

IX

;

where in general formula II to general formula IX, $R_1$ is hydrogen or methyl, and $R_2$ is oxygen or imino;
formula II is double bond-containing acrylic acid;
formula III is double bond-containing polyethylene glycol, where m represents number of polyethylene glycol arms, which is an integer of 2 to 8; n represents number of repeating units of polyethylene glycol, which is an integer of 28 to 112, and corresponds to formula III having a molecular weight of 2 to 40 kDa;
in formula III, "●" represents a carbon atom located in middle of a multi-arm polyethylene glycol acrylate or multi-arm polyethylene glycol methacrylate and used to connect multiple identical polyethylene glycol acrylate or polyethylene glycol methacrylate chain segments;
the double bond-containing polyethylene glycol represented by formula III includes at least one of polyethylene glycol diacrylate, eight-arm polyethylene glycol acrylate, and four-arm polyethylene glycol acrylate.
formula IV is double bond-containing hyaluronic acid, where n represents number of repeating units of hyaluronic acid, which is an integer of 25 to 2500, and corresponds to formula IV having a molecular weight of 10 to 1000 kDa;
formula V is double bond-containing gelatin;
formula VI is double bond-containing chitosan, where x, y, and z represent number of respective repeating units of chitosan, which is an integer of 62 to 6200, and corresponds to formula VI having a molecular weight of 10 to 1000 kDa;
in formula VII, $R_3$ is selected from hydrogen, $-CH_3$, -OH, $-CH_2-COOH$, $-CH_2CH_3$, $-CH_2-CH(CH_3)_2$, $-CH_2-C_6H_5$, $-CH_2-C_6H_4-OH$, or $-CH_2-SH$, and
the components for preparing the hemostatic sponge further comprise a salt-containing aqueous solution, namely, a salt solution.

2. The hemostatic sponge according to claim 1, **characterized in that** the components for preparing the hemostatic sponge comprise, by mass, 3 to 20 parts of the double bond-containing compound, 1 to 10 parts of the chitosan, and 0.7 to 15 parts of the photoinitiator.

3. The hemostatic sponge according to claim 1, **characterized in that** the chitosan is a water-soluble chitosan.

4. The hemostatic sponge according to claim 1, **characterized in that** the chitosan has a molecular weight of 5 to 20 kDa.

5. The hemostatic sponge according to claim 1, **characterized in that** the photoinitiator includes at least one of photoinitiator 651, photoinitiator 1173, photoinitiator 2959, TPO, $\alpha$-ketoglutaric acid, and LAP.

6. The hemostatic sponge according to claim 1, **characterized in that** the salt includes at least one of NaCl, $CaCl_2$, KCl, $NaH_2PO_4$, $KH_2PO_4$, $Na_2CO_3$, $Na_2SO_4$, $Na_2HPO_4$, $K_2HPO_4$, $Na_3Cit$, and $Na_3PO_4$.

7. The hemostatic sponge according to claim 1, **characterized in that** the salt solution is a saturated aqueous solution.

8. The hemostatic sponge according to claim 1, **characterized in that** a concentration of the salt solution is 15wt% to 40wt%.

9. The hemostatic sponge according to claim 1, **characterized in that** the salt solution is a PBS buffer solution.

10. A method for preparing the hemostatic sponge according to any one of claims 1-9, **characterized in that** the method comprises:

a precursor solution preparation step, comprising mixing the components according to respective formula amounts, and dissolving same in a solvent to prepare a precursor solution;
a stirring step, comprising vigorously stirring the precursor solution such that the precursor solution contains uniform bubbles;
a depressurizing treatment step, comprising performing depressurizing treatment on the precursor solution to obtain an expanded precursor solution;
an illumination step, comprising illuminating the expanded precursor solution to obtain a formed sponge;
a soaking step, comprising soaking the formed sponge in a salt solution to obtain a soaked sponge; and
a freeze-drying step, comprising freeze-drying the soaked sponge to obtain the hemostatic sponge.

11. The preparation method according to claim 10, **characterized in that** in the precursor solution preparation step, the solvent is water.

**12.** The preparation method according to claim 10, **characterized in that** in the precursor solution preparation step, a mass of the solvent is 1 to 100 times a mass of the chitosan.

**13.** The preparation method according to claim 12, **characterized in that** in the precursor solution preparation step, the mass of the solvent is 10 to 100 times the mass of the chitosan.

**14.** The preparation method according to claim 10, **characterized in that** in the precursor solution preparation step, the precursor solution is stirred until uniform bubbles form, prior to performing the depressurizing treatment step.

**15.** The preparation method according to claim 10, **characterized in that** in the depressurizing treatment step, a vacuum degree in a container containing the precursor solution is 20 to 100 mbar.

**16.** The preparation method according to claim 10, **characterized in that** in the illumination step, a wavelength of a light used in illumination is 200 to 450 nm.

**17.** The preparation method according to claim 10, **characterized in that** in the illumination step, a duration of illumination is 1 to 100 min.

**18.** The preparation method according to claim 10, **characterized in that** in the soaking step, the salt solution includes at least one of NaCl aqueous solution, $CaCl_2$ aqueous solution, KCl aqueous solution, $NaH_2PO_4$ aqueous solution, $KH_2PO_4$ aqueous solution, $Na_2CO_3$ aqueous solution, $Na_2SO_4$ aqueous solution, $Na_2HPO_4$ aqueous solution, $K_2HPO_4$ aqueous solution, $Na_3Cit$ aqueous solution, $Na_3PO_4$ aqueous solution, and PBS buffer solution.

**19.** The preparation method according to claim 10, **characterized in that** in the soaking step, the salt solution is a saturated aqueous solution.

**20.** The preparation method according to claim 10, **characterized in that** in the soaking step, a concentration of the salt solution is 15wt% to 40wt%.

**21.** The preparation method according to claim 10, **characterized in that** in the soaking step, the salt solution is a PBS buffer solution.

**22.** The preparation method according to claim 10, **characterized in that** in the soaking step, the duration of soaking in the salt solution is 1 to 200 min.

**23.** The preparation method according to claim 10, **characterized in that** in the soaking step, after soaking in the salt solution is completed, soaking is performed using water prior to performing the freeze-drying step.

**24.** The preparation method according to claim 10, **characterized in that** in the soaking step, after soaking in the salt solution is completed, the sponge is taken out of the salt solution, allowed to stand for 1 to 100 h, and is then soaked using water.

**25.** The preparation method according to claim 10, **characterized in that** in the soaking step, a duration of soaking in water is 1 to 100 h.

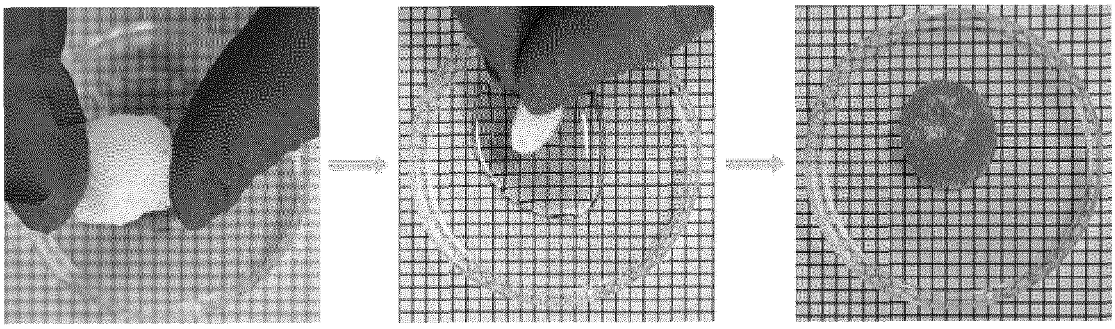

Fig. 1

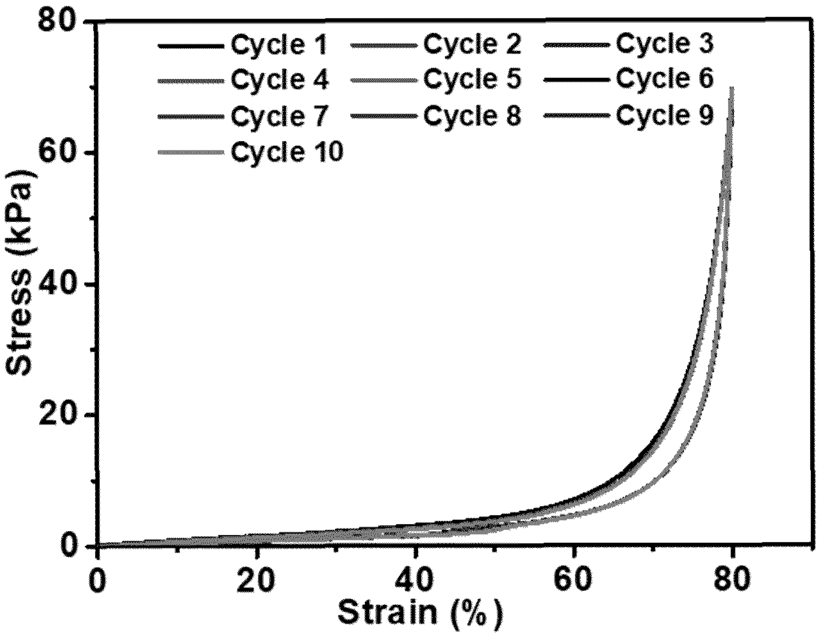
Cycle 1
Cycle 2
Cycle 3
Cycle 4
Cycle 5
Cycle 6
Cycle 7
Cycle 8
Cycle 9
Cycle 10
Stress (kPa)
Strain (%)
0
20
40
60
80

Fig. 2

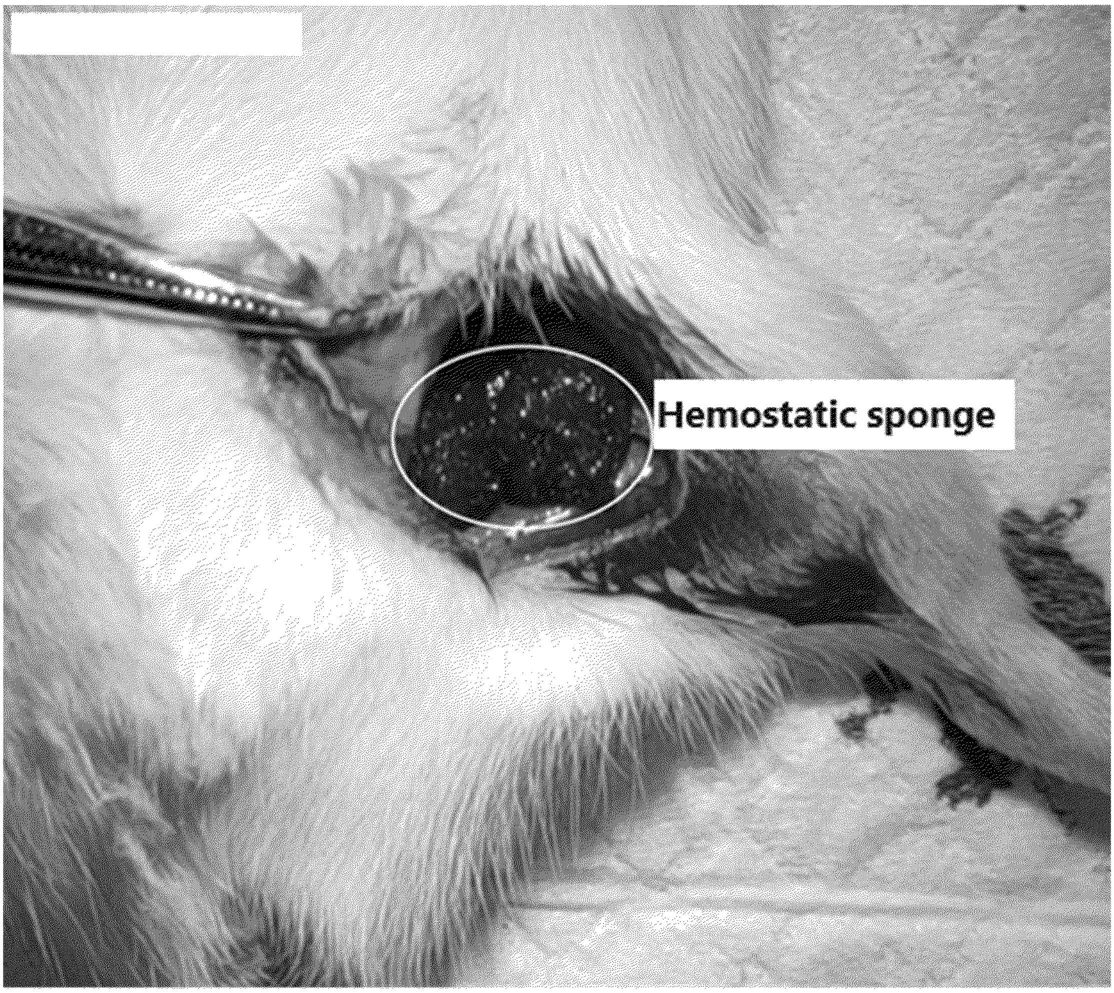


Fig. 3

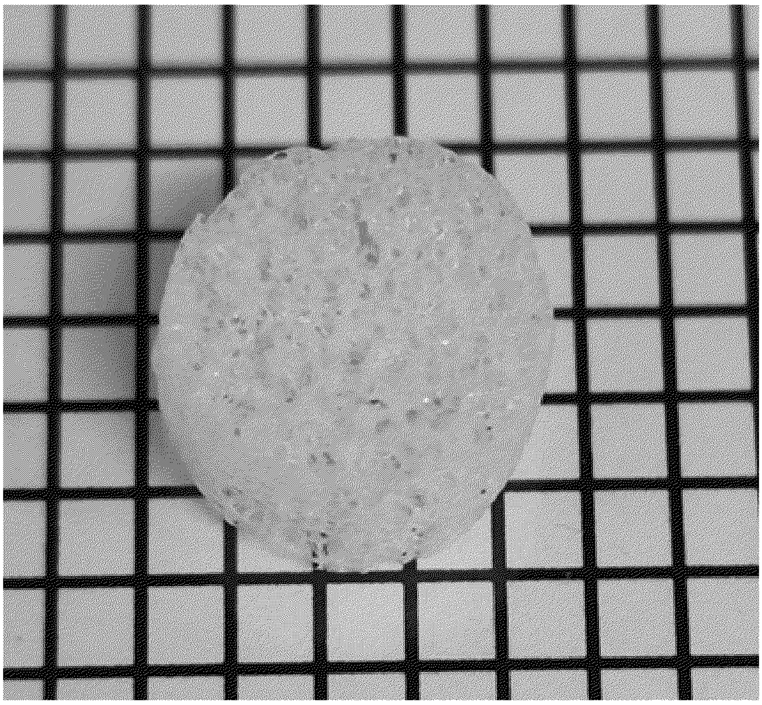

Fig. 4

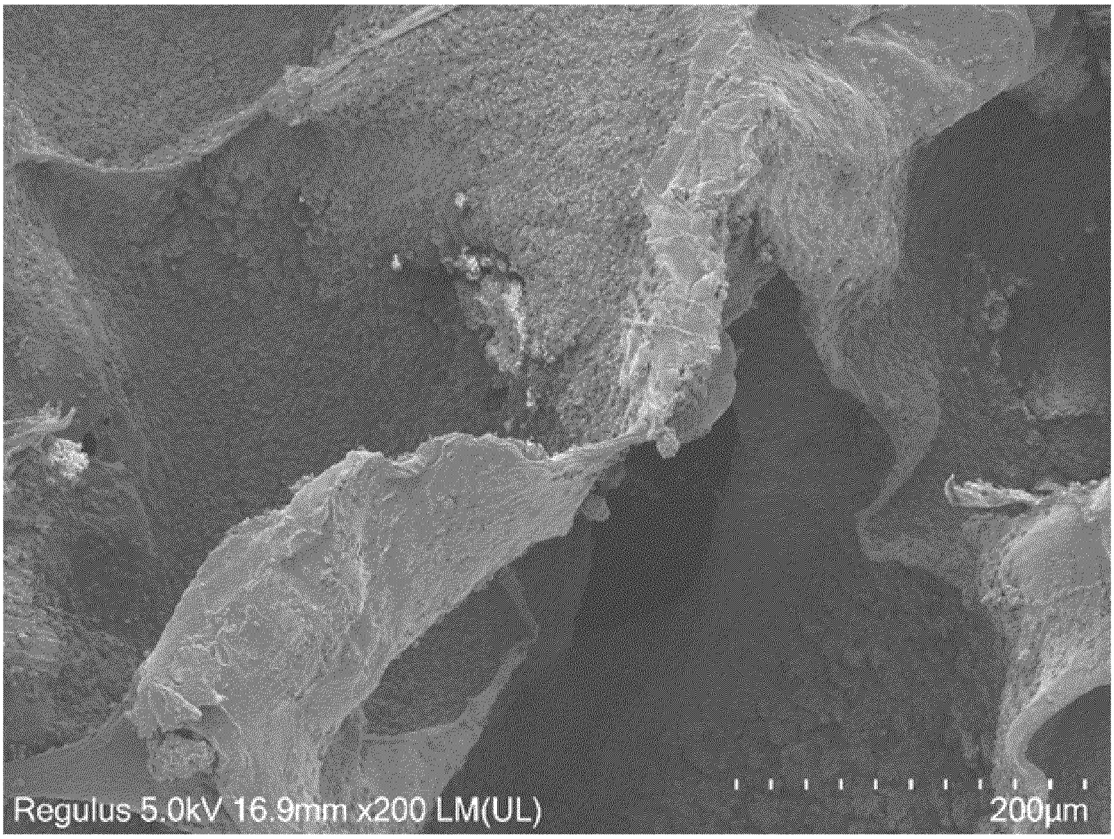


Fig. 5

**INTERNATIONAL SEARCH REPORT**

International application No.
**PCT/CN2022/138355**

**A. CLASSIFICATION OF SUBJECT MATTER**

A61L 24/00(2006.01)i;A61L 24/06(2006.01)i;A61L 24/08(2006.01)i;C08F 251/00(2006.01)i;C08F 222/20(2006.01)i;C08F 220/58(2006.01)i;C08F 2/48(2006.01)i;C08F 299/00(2006.01)i;C08G 81/00(2006.01)i

According to International Patent Classification (IPC) or to both national classification and IPC

**B. FIELDS SEARCHED**

Minimum documentation searched (classification system followed by classification symbols)

A61L(2006.01), C08F(2006.01)

Documentation searched other than minimum documentation to the extent that such documents are included in the fields searched

Electronic data base consulted during the international search (name of data base and, where practicable, search terms used)

CNABS; CNTXT; CNKI; SIPOABS; EPTXT; BRTXT; USTXT; WOTXT; Elsevier; ISI Web of Science: 丙烯酸, 双键, 透明质酸, 玻尿酸, 聚乙二醇, 明胶, 壳聚糖, 壳多糖, 几丁糖, 几丁质, 氨酸, 光引发剂, 盐, 氯化钠, 钠盐, 钙盐, 镁盐, 止血, 海绵, 多孔, acrylic acid, Acrylat+, double bond, hyaluronic acid, HA, polyethylene glycol, PEG, gelatin, chitosan, ammonia, photoinitiator, salt, sodium chloride, sodium salt, calcium salt, magnesium salt, hemostasis, sponge, porous

**C. DOCUMENTS CONSIDERED TO BE RELEVANT**

| Category* | Citation of document, with indication, where appropriate, of the relevant passages | Relevant to claim No. |
|---|---|---|
| X | CN 111116973 A (UNIVERSITY OF CHINESE ACADEMY OF SCIENCES, WENZHOU INSTITUTE, UCAS (WENZHOU INSTITUTE OF BIOMATERIALS AND ENGINEERING)) 08 May 2020 (2020-05-08)<br>description, paragraphs 6-16, and claims 1-19 | 1-25 |
| X | CN 107383290 A (CHENGDU MEDART MEDICAL SCIENTIFIC CO., LTD.) 24 November 2017 (2017-11-24)<br>description, paragraphs 6-16, and claims 1-8 | 1-25 |
| PX | CN 114470305 A (SOUTHERN UNIVERSITY OF SCIENCE AND TECHNOLOGY) 13 May 2022 (2022-05-13)<br>claims 1-10, and embodiment 1 | 1-25 |
| A | CN 109091705 A (LYU YANG et al.) 28 December 2018 (2018-12-28)<br>entire document | 1-25 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 February 2023** | **04 March 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

International application No.
**PCT/CN2022/138355**

**C. DOCUMENTS CONSIDERED TO BE RELEVANT**

| Category* | Citation of document, with indication, where appropriate, of the relevant passages | Relevant to claim No. |
|---|---|---|
| A | CN 113730640 A (SUZHOU YONGQINQUAN INTELLIGENT EQUIPMENT CO., LTD.) 03 December 2021 (2021-12-03) entire document | 1-25 |
| A | CN 104027833 A (WUHAN TEXTILE UNIVERSITY) 10 September 2014 (2014-09-10) entire document | 1-25 |
| A | CN 108187130 A (ZHEJIANG UNIVERSITY) 22 June 2018 (2018-06-22) entire document | 1-25 |
| A | CN 108495658 A (BAXTER INTERNATIONAL INC.) 04 September 2018 (2018-09-04) entire document | 1-25 |
| A | EP 3103485 A1 (COMMISSARIAT ENERGIE ATOMIQUE et al.) 14 December 2016 (2016-12-14) entire document | 1-25 |
| A | US 2013230493 A1 (ALSBERG, E. et al.) 05 September 2013 (2013-09-05) entire document | 1-25 |
| A | US 2020179562 A1 (COLLPLANT LTD.) 11 June 2020 (2020-06-11) entire document | 1-25 |
| A | WO 2009134414 A2 (THE BOARD OF TRUSTEES OF THE LELAND STANFORD JUNIOR UNIVERSITY et al.) 05 November 2009 (2009-11-05) entire document | 1-25 |

Form PCT/ISA/210 (second sheet) (July 2022)

**INTERNATIONAL SEARCH REPORT**
**Information on patent family members**

International application No.
**PCT/CN2022/138355**

| Patent document cited in search report | Publication date (day/month/year) | Patent family member(s) | Publication date (day/month/year) |
|---|---|---|---|
| CN 111116973 A | 08 May 2020 | None | |
| CN 107383290 A | 24 November 2017 | None | |
| CN 114470305 A | 13 May 2022 | None | |
| CN 109091705 A | 28 December 2018 | None | |
| CN 113730640 A | 03 December 2021 | None | |
| CN 104027833 A | 10 September 2014 | None | |
| CN 108187130 A | 22 June 2018 | WO 2019052171 A1 | 21 March 2019 |
| | | KR 20200049781 A | 08 May 2020 |
| | | KR 102469682 B1 | 21 November 2022 |
| | | EP 3681546 A1 | 22 July 2020 |
| | | EP 3681546 A4 | 16 June 2021 |
| | | US 2020206383 A1 | 02 July 2020 |
| | | US 11446409 B2 | 20 September 2022 |
| | | JP 2021500193 A | 07 January 2021 |
| | | JP 6893074 B2 | 23 June 2021 |
| CN 108495658 A | 04 September 2018 | JP 2018527082 A | 20 September 2018 |
| | | JP 6877409 B2 | 26 May 2021 |
| | | US 2018243465 A1 | 30 August 2018 |
| | | BR 112018004056 A2 | 02 October 2018 |
| | | BR 112018004056 B1 | 29 June 2021 |
| | | JP 2021087867 A | 10 June 2021 |
| | | CA 2996853 A1 | 09 March 2017 |
| | | EP 3943127 A1 | 26 January 2022 |
| | | NZ 740264 A | 29 October 2021 |
| | | AU 2016313773 A1 | 22 March 2018 |
| | | AU 2016313773 B2 | 26 November 2020 |
| | | EP 3344299 A1 | 11 July 2018 |
| | | EP 3344299 B1 | 11 August 2021 |
| | | ES 2897772 T3 | 02 March 2022 |
| | | KR 20180048831 A | 10 May 2018 |
| | | WO 2017037178 A1 | 09 March 2017 |
| | | MX 2018002649 A | 20 June 2018 |
| EP 3103485 A1 | 14 December 2016 | EP 3307337 A1 | 18 April 2018 |
| | | WO 2016198238 A1 | 15 December 2016 |
| US 2013230493 A1 | 05 September 2013 | US 2018064815 A1 | 08 March 2018 |
| | | US 9642914 B2 | 09 May 2017 |
| | | US 2011008443 A1 | 13 January 2011 |
| | | US 8273373 B2 | 25 September 2012 |
| US 2020179562 A1 | 11 June 2020 | EP 3634512 A1 | 15 April 2020 |
| | | EP 3634512 A4 | 14 April 2021 |
| | | WO 2018225076 A1 | 13 December 2018 |
| | | WO 2018225076 A8 | 28 February 2019 |
| | | AU 2018282131 A1 | 30 January 2020 |
| | | CA 3065481 A1 | 13 December 2018 |
| | | JP 2020522357 A | 30 July 2020 |
| WO 2009134414 A2 | 05 November 2009 | US 2009280182 A1 | 12 November 2009 |
| | | WO 2009134414 A3 | 29 July 2010 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *CHEMICAL ABSTRACTS*, 24650-42-8
- *CHEMICAL ABSTRACTS*, 106797-53-9
- *CHEMICAL ABSTRACTS*, 75980-60-8
- *CHEMICAL ABSTRACTS*, 328-50-7
- *CHEMICAL ABSTRACTS*, 85073-19-4